# EUROPEAN PATENT APPLICATION

(11) **EP 4 512 413 A1**
(43) Date of publication of application: **26.02.2025**
(21) Application number: 24768467.3
(22) Date of filing: 25.06.2024
(51) Int. Cl.: A61K 38/20, A61K 48/00, A61P 25/28

(54) **USE OF IL-27 PROTEIN IN PREPARATION OF PRODUCT FOR TREATING AND/OR DELAYING ALZHEIMER'S DISEASE**

(30) Priority: 30.06.2023 CN 202310798677
(71) Applicant: Guangdong Jiantebo Biotechnology Co., Ltd., Zhongshan, Guangdong 528400 (CN)
(72) Inventor: ZHANG, Li, Zhongshan, Guangdong 528400 (CN); WANG, Qian, Zhongshan, Guangdong 528400 (CN); WEI, JiAn, Zhongshan, Guangdong 528400 (CN); ZHENG, Pei, Zhongshan, Guangdong 528400 (CN); YIN, Zhinan, Zhongshan, Guangdong 528400 (CN); YANG, Hengwen, Zhongshan, Guangdong 528400 (CN)
(74) Representative: Bryn Aarflot AS
(86) International application number: PCT/CN2024/101232
(87) International publication number: WO 2025/002100

(57) **Abstract**

Use of an interleukin 27 (IL-27) protein in preparation of a product for treating and/or delaying Alzheimer's disease (AD) is provided, belonging to the technical field of biomedicine. The IL-27 protein refers to a recombinant IL-27 protein targeting a therapeutic target II,-27, and is selected from the group consisting of a mouse-derived IL-27 protein and a human-derived IL-27 protein, as well as a mammalian IL-27 protein other than the mouse-derived IL-27 protein and the human-derived IL-27 protein. The recombinant IL-27 protein can effectively alleviate the AD caused by Aβ deposition, and can selectively bind to a target receptor, thereby ensuring an accuracy of test results. The protein receptor is highly expressed in the dentate gyrus region of hippocampus, and guarantees drug targeting to the greatest extent. The recombinant IL-27 protein also shows desirable application prospects, can quickly and effectively improve and alleviate behaviors such as the memory impairment in mice with AD, and has a clinical transformation value.

## Description

The present application claims priority to the Chinese Patent Application No. 202310798677.3, filed with the China National Intellectual Property Administration (CNIPA) on June 30, 2023, and entitled "USE OF INTERLEUKIN 27 PROTEIN IN PREPARATION OF PRODUCT FOR TREATING AND/OR DELAYING ALZHEIMER'S DISEASE", which is incorporated herein by reference in its entirety.

### TECHNICAL FIELD

The present disclosure belongs to the technical field of biomedicine, and in particular relates to use of an interleukin 27 (IL-27) protein in preparation of a product for treating and/or delaying Alzheimer's disease (AD).

### BACKGROUND

Alzheimer's disease (AD) seriously affects human health. As the average human life expectancy increases and the aging society becomes more serious, the prevalence of AD is also increasing. AD patients generally show behavioral manifestations such as memory loss and weakened learning ability, which can greatly affect the development of individuals, families, and even society. As a neurodegenerative disease with high disability and mortality rates, the AD has caused serious social and family burdens.

The main pathological feature of AD is the degenerative death of neurons in the hippocampus and other brain regions with behavioral manifestations of learning and cognitive dysfunction. Synapses are key structures for neurotransmission and neuroplasticity, and are the basis for learning, memory, and cognitive function. The learning and memory process dominated by hippocampal neurons participates in the learning of various behavioral scenarios such as spatial memory, working memory through neural signal transmission and neuroplasticity regulation by synaptic structure and functional plasticity. At present, the pathological molecular mechanism of AD is still unclear. There are many theories, such as the classic amyloid protein (Aβ) deposition and neurofibrillary tangles (NFTs), or Tau protein phosphorylation, thus affecting neuronal function and survival. However, in recent years, most clinical trials on the development of targeted drugs for Aβ and other molecules have failed, suggesting the possible multiple pathogenesis of AD and the urgent need for new drug therapeutic targets.

Interleukin 27 (IL-27) is composed of two subunits, EBI-3 and p28, and is mainly secreted by dendritic cells (DCs) and macrophages. An IL-27 receptor includes two subunits, gp130 and IL-27R (also known as TCCR or WSX1), and is mainly expressed on immune cells such as T cells, B cells, NK cells, and myeloid cells.

IL-27 has a bidirectional regulatory effect on immune response. On one hand, IL-27 can promote the differentiation of naive CD4+T cells to Th1: in viral infection, the IL-27 induces excessive activation of CD4+T to Th1 and IFN-production, thereby leading to the death of mice. On the other hand, the IL-27 can also promote T cells to express inhibitory molecules such as SOCSs, directly inhibiting the differentiation and function of Th2 and Th17 cells. In addition, the IL-27 can also limit the production of IL-2 and granulocyte macrophage-colony stimulating factor (GM-CSF), induce IL-10 and promote the function of Treg cell subsets, and indirectly inhibit the inflammatory response mediated by Th17 and Th1 cells.

In summary, IL-27 currently receives extensive attention in the differentiation and function of immune cells, but the function of IL-27 in AD still remains unknown.

### SUMMARY

In order to solve the above problems, the present disclosure provides use of an IL-27 protein in preparation of a product for treating and/or delaying AD, where the IL-27 protein is a recombinant IL-27 protein targeting a therapeutic target II,-27, comprising a mouse-derived IL-27 protein and a human-derived IL-27 protein, as well as a mammalian IL-27 protein other than the mouse-derived IL-27 protein and the human-derived IL-27 protein.

Preferably, the recombinant IL-27 protein is any one selected from the group consisting of:
A, a protein with an amino acid sequence set forth in SEQ ID NO: 1; and
B, a protein derived from the amino acid sequence set forth in SEQ ID NO: 1 or a mammalian homologous protein sequence thereof, where the protein is prepared by subjecting the amino acid sequence set forth in SEQ ID NO: 1 to substitution, deletion, or addition of one or more amino acid residues and has a same activity.

Preferably, a gene encoding the recombinant IL-27 protein includes:
a nucleotide sequence set forth in SEQ ID NO: 2; alternatively, a nucleotide sequence encoding a protein with a same activity and prepared by subjecting the nucleotide sequence set forth in SEQ ID NO: 2 to substitution, deletion, and/or addition of one or more nucleotides; alternatively, a nucleotide sequence encoding the amino acid sequence set forth in SEQ ID NO: 1.

Preferably, a recombinant expression plasmid, a viral vector, or an mRNA fragment based on the recombinant IL-27 protein has any one of the following features:
A, including the recombinant IL-27 protein;
B, including a nucleotide sequence encoding the recombinant IL-27 protein; and
C, including a fragment with a nucleotide sequence encoding the recombinant IL-27 protein.

Preferably, a liposome preparation of the recombinant expression plasmid, the viral vector, or the mRNA fragment expressing the recombinant IL-27 protein is prepared by subjecting a nucleic acid preparation encoding the recombinant IL-27 protein to liposome modification.

Preferably, the recombinant expression plasmid or the viral vector is used to express the recombinant IL-27 protein in *Escherichia coli,* an insect cell, and a mammalian cell and *in vivo.*

Preferably, a host cell is based on the recombinant IL-27 protein; and the host cell includes the recombinant expression plasmid.

Preferably, a cloned plasmid is based on the recombinant IL-27 protein; and the cloned plasmid has any one of the following features:
A, including the recombinant IL-27 protein;
B, including a nucleotide sequence encoding the recombinant IL-27 protein; and
C, including a fragment with a nucleotide sequence encoding the recombinant IL-27 protein.

Preferably, the product is any one selected from the group consisting of a drug, a detection reagent, a kit, and a diagnostic reagent.

Preferably, the drug includes the IL-27 protein and a pharmaceutically acceptable carrier;
the pharmaceutically acceptable carrier is at least one selected from the group consisting of a sustained-release agent, an excipient, a filler, a binder, a wetting agent, a disintegrant, an absorption promoter, a surfactant, and a lubricant; and
a dosage form of the drug is selected from the group consisting of an ointment, a powder, a tablet, a capsule, and a granule.

The present disclosure provides use of an IL-27 protein in preparation of a product for treating and/or delaying AD is provided, belonging to the technical field of biomedicine. The recombinant IL-27 protein includes: a protein with an amino acid sequence set forth in SEQ ID NO: 1; and a protein derived from the amino acid sequence set forth in SEQ ID NO: 1, where the protein is prepared by subjecting the amino acid sequence set in SEQ ID NO: 1 to substitution, deletion, or addition of one or more amino acid residues and has a same activity. The recombinant IL-27 protein targets a novel therapeutic target RmII,-27, can effectively alleviate AD caused by Aβ deposition, and provides a new approach for the treatment and alleviation of AD.

In summary, the recombinant IL-27 protein of the present disclosure can effectively delay and treat AD-induced memory impairment. This receptor protein has the following beneficial effects:
(1) the IL-27 can selectively and specifically bind to its receptor, thereby ensuring the accuracy of detection results;
(2) this protein receptor is highly expressed specifically in the dentate gyrus region of hippocampus, and ensures drug targeting to the greatest extent; and
(3) the recombinant IL-27 protein shows desirable application prospects, can quickly and effectively improve and alleviate behaviors such as the memory impairment in mice with AD, and has an extremely important clinical transformation value.

### BRIEF DESCRIPTION OF THE DRAWINGS

FIG. 1 shows an immunohistochemical staining image (DAPI) of the C57BL/6J experimental mice in Example 1 of the present disclosure for *in situ* hybridization detection of an IL-27 receptor gene;
FIG. 2 shows an immunohistochemical staining image (Vgat) of the C57BL/6J experimental mice in Example 1 of the present disclosure for *in situ* hybridization detection of an IL-27 receptor gene;
FIG. 3 shows an immunohistochemical staining image (IL27Ra) of the C57BL/6J experimental mice in Example 1 of the present disclosure for *in situ* hybridization detection of an IL-27 receptor gene;
FIG. 4 shows an immunohistochemical staining image (VGlut) of the C57BL/6J experimental mice in Example 1 of the present disclosure for *in situ* hybridization detection of an IL-27 receptor gene;
FIG. 5 shows an immunohistochemical staining image (Merge) of the C57BL/6J experimental mice in Example 1 of the present disclosure for *in situ* hybridization detection of an IL-27 receptor gene;
FIG. 6 shows a comparison of discrimination indexes in a novel object recognition (NOR) test of gene-deficient mice in Example 2 of the present disclosure;
FIG. 7 shows a comparison of discrimination indexes in a novel object location (NOL) test of gene-deficient mice in Example 2 of the present disclosure;
FIG. 8 shows a latency time of the gene-deficient mice to reach a target hole in a Barnes maze test in Example 2 of the present disclosure;
FIG. 9 shows a number of gene-deficient mice entering wrong holes in the Barnes maze test in Example 2 of the present disclosure;
FIG. 10 shows a comparison of discrimination indexes of FAD5X AD (FAD) experimental mice (RM IL-27 group and control group) in the novel object recognition (NOR) test at a 2 h time point in Example 3 of the present disclosure;
FIG. 11 shows a comparison of discrimination indexes of FADSX AD (FAD) experimental mice (RM IL-27 group and control group) in the novel object location (NOR) test at a 2 h time point in Example 3 of the present disclosure;
FIG. 12 shows a comparison of discrimination indexes of FAD5X AD (FAD) experimental mice (RM IL-27 group and control group) in the novel object recognition(NOR) test at a 24 h time point in Example 3 of the present disclosure;
FIG. 13 shows a comparison of discrimination indexes of FAD5X AD (FAD) experimental mice (RM IL-27 group and control group) in the novel object location(NOR) test at a 24 h time point in Example 3 of the present disclosure;
FIG. 14 shows a latency time of the FAD5X AD (FAD) experimental mice (RM IL-27 group and control group) to reach a target hole in a Barnes maze test in Example 3 of the present disclosure;
FIG. 15 shows a comparison of discrimination indexes of FADSX AD (FAD) experimental mice (mRNA-IL-27 group and control group) in the NOR test at 36 h after drug administration and at 72 h after drug administration in Example 4 of the present disclosure;
FIG. 16 shows a comparison of discrimination indexes of FAD5X AD (FAD) experimental mice (mRNA-IL-27 group and control group) in the NOL test at 36 h after drug administration and at 72 h after drug administration in Example 4 of the present disclosure; and
FIG. 17 shows a latency time of the FAD5X AD (FAD) experimental mice (mRNA-IL-27 group and control group) to reach a target hole in a Barnes maze test in Example 4 of the present disclosure.

The implementation of the objective, functional characteristics and advantages of the present disclosure will be further described in conjunction with the examples and with reference to the drawings.

### DETAILED DESCRIPTION OF THE EMBODIMENTS

The following clearly and completely describes the technical solutions of the present disclosure with reference to the example. Apparently, the described example is merely a part rather than all of the examples of the present disclosure. All other embodiments obtained by those skilled in the art based on the embodiments of the present disclosure without creative efforts shall fall within the protection scope of the present disclosure.

Unless otherwise defined below, all technical and scientific terms used in the detailed description of the present disclosure are intended to have the same meanings as commonly understood by those skilled in the art. While it is believed that the following terms are well understood by those skilled in the art, the following definitions are nevertheless set forth to better explain the present disclosure.

As used in the present disclosure, the terms "comprising", "including", "having", "containing", or "involving" are inclusive or open-ended and do not exclude other unrecited elements or method steps. The term "consisting of" is considered to be a preferred embodiment of the term "comprising". If hereinafter a group is defined as comprising at least a certain number of embodiments, this shall also be understood to disclose a group which preferably consists only of these embodiments.

When referring to a singular noun, an indefinite or definite article e.g. "a" or "an", "the", include a plural of that noun.

The following is provided merely to aid understanding of the present disclosure. These definitions should not be construed to have a scope less than that understood by one skilled in the art.

The technical solution of the present disclosure is further described in detail below in combination with specific examples, but does not constitute any limitation to the present disclosure. Any limited number of modifications made by anyone within the scope of the claims of the present disclosure are still within the scope of the claims of the present disclosure.

The present disclosure provides use of an IL-27 protein in preparation of a product for treating and/or delaying AD, where the IL-27 protein is a recombinant IL-27 protein targeting a therapeutic target II,-27, and is selected from the group consisting of a mouse-derived IL-27 protein and a human-derived IL-27 protein, as well as a mammalian IL-27 protein other than the mouse-derived IL-27 protein and the human-derived IL-27 protein.

In the present disclosure, the use includes treating the AD and alleviating the symptoms of patients.

Further, the recombinant IL-27 protein is any one selected from the group consisting of:
A, a protein with an amino acid sequence set forth in SEQ ID NO: 1; and
B, a protein derived from the amino acid sequence set forth in SEQ ID NO: 1 or a mammalian homologous protein sequence thereof, where the protein is prepared by subjecting the amino acid sequence set forth in SEQ ID NO: 1 to substitution, deletion, or addition of one or more amino acid residues and has a same activity.

The recombinant IL-27 protein may include either A or B.

In the present disclosure, the amino acid sequence set forth in SEQ ID NO: 1 is specifically as follows: MGQVTGDLGWRLSLLLLPLLLVQAGSWGFPTDPLSLQELRREFTVSLYLARKLLSEVQGYV HSFAESRLPGVNLDLLPLGYHLPNVSLTFQAWHHLSDSERLCFLATTLRPFPAMLGGLGTQG TWTSSEREQLWAMRLDLRDLHRHLRFQVLAAGFKCSKEEEDKEEEEEEEEEEKKLPLGAL GGPNQVSSQVSWPQLLYTYQLLHSLELVLSRAVRDLLLLSLPRRPGSAWDS.

The IL-27 protein includes two subunits, EBI-3EBI3 (NCBI Reference Sequence: NM_015766) and P28 (NCBI Reference Sequence: NM_145636), and SEQ ID NO: 1 is an amino acid sequence of the P28 subunit.

In the present disclosure, the IL-27 protein, i.e., the recombinant IL-27 protein, aims to the novel therapeutic target II,-27, including mouse-derived IL-27, human-derived II,-27, and IL-27 derived from other mammalian. The recombinant IL-27 protein can effectively alleviate AD caused by Aβ deposition, and the IL-27 can selectively and specifically bind to its receptor, thereby ensuring the accuracy of test results. This recombinant IL-27 protein receptor is highly expressed in the dentate gyrus region of hippocampus, thus guaranteeing the drug targeting to the greatest extent. The recombinant IL-27 protein has a desirable application prospect, can quickly and effectively improve and alleviate behaviors such as the memory impairment in mice with AD, and has an extremely important clinical transformation value. In conclusion, the recombinant IL-27 protein provides a new approach for the treatment and alleviation of AD.

Further, a gene encoding the recombinant IL-27 protein includes any one of the following nucleotide sequences:
(1) a nucleotide sequence set forth in SEQ ID NO: 2; alternatively,
(2) a nucleotide sequence encoding a protein with a same activity and prepared by subjecting the nucleotide sequence set forth in SEQ ID NO: 2 to substitution, deletion, and/or addition of one or more nucleotides; alternatively,
(3) a nucleotide sequence encoding the amino acid sequence set forth in SEQ ID NO: 1.

In the present disclosure, the nucleotide sequence set forth in SEQ ID NO: 2 is specifically as follows:
cttagtgaacacaaagctgaaagtacaagtaggacagaaagtgaaactgggcgcagcccccagtataagacccccctacccagg agatggctgcacacagaggctgggccctgacatgggccaggtgacaggagaccttggctggcgtgagttggaagcggcgactgggctgag ggctgggaggtttggtggagcccaggagaggttgggaaagtttgacaggtagggttggggcagaaagttgaggggcaacacgttggccacg tagacaggcatggctagctgaccacgggaatgagactagtgtaagtgggcaggggttagagcagtggaattatggttggctgggtaagtgata gcagtgtctgggatgtgtcaggaggactggacaggcaacctggccaggagcaggactaaacagacaaatgaagagtgtagagggaagagg ctgagaaccgaggacagtcagaggaacggcacaggggagctggtaagagcggtgactgggaccaagggggcaacctctctgggtaaaggt ctctaacttacccaaggtctggaacttagaaggttgcccagtctgcagctgggagctgaaggactcggaacagagttgtaggaagaggggcaa gttgtaggctgggtaggctcaagggctggagcatgagggcgggattctctacaaaggggtcttggggtgtctagaattttgagaggtggtaagt gggtatgagctggcacaccttagtctgggatgtaatatttaccaaggcctaagggttcatgccttctccccatcctagagctaggaagaattctattt agtctccaggatattagggagtcagaatggttctcaagccaagaaacctgaatgccagagatctcaatcactgcccacttctgaaagtacatggg gaaaatagatgtcgtcccccacgcccccaagctttcattaactactgctgagggcactttagggaggtttatagattatgaatggatagaagggac ctggagaggagcaagggtggggagagacttggggatagttttggtttgggtttcggggagaattgtgtaactcccgcttgagagagaaaccact gaagattgccttcttcccagagctcccacttcacagccctgctccctcttactacaaattgcagggcttgaagtcccctgaaaagtcagatagcatg actctcactccacagctgggagcctgggacacaaaaataagaggtgacctgctcaaggcaggatgaagacactagcctccccctccatcagtg 
ccatccccaccacatgtcccttccaggaccatactgctaatttaccctagaagggctcagtgcacagtggcatattttccatatgagtccctagaat
agaaccactggctttgccaggtcttccctagggccctaggtctgtttaaagcctcagggctgtctgggtgaacccaaggaatccttggcagaaca
cagtgttgccacctctggagcaggtgggttcttcctctgagttccctggggatagagtactggcctgtgtttgggtggatctggagtccaaccctg
cctccctgacctctcactgactaaccgtggctagtcactttacccagtctcaagggctagggcacacacatgcagttatgtaggctgggcactgg
aaaaggcaattgactaagggatctagttggagtgtgtgtattaagatcctgcttttggggcttggaggtggtagcacacacctttaatcccagcact
tgggaggcagaggcaggcagatttctgagtacaaggccagcctggtctacagagtgagttccaggacagccatggctacacagagaaaccct
gtctagaaaacaaaacgaaacaacaacaacaacaacaaagatcctgcctttggtgttcttacaacagtgtgcagagtgacatgggggtagcctct
gtggtccaaatgatggttctaagagggtagcttgaagatgccaaagagactcagtgcttgtatagatcggtttcttccttctccagggttctccagcc
tttatcttgtcacctttcctcctttctccagggctcagcctgttgctgctacccttgcttctggtacaagctggttcctgggggttcccaacagaccccc
tgagccttcaagagctgcgcagggaattcacagtcagcctgtaccttgccaggaagctgctctctgaggttcagggctatgtccacagctttgtga
gtctccttagcgcaattgggtctggaggtgaggacacccccaagtcccactggccctcactaacagaatttaaccctcaggctgaatctcgattg
ccaggagtgaacctggacctcctgcccctgggataccatcttcccaatgtttccctgactttccaggcatggcatcacctctctgtaagtgaacgg
caaggggatggggagactggcagtaaagtatggtacatagacaagtgtttgagcagatgtggatctgcaggtttgtatggctaatatgtctctgta
tgtaaaggtgaagctggaagctgagggtagtggtacacatctgtaatcccaatacttgggaggcagagactaggatttgaggaagatagagga
acaaaggagagggagctgggatttgaaagtggggaccgcaagatggtgagcatgacactgggggaagaggtgggactgggaatggaggta
gctatgggaaagagatgggctgacgactgtgatgggagtggggtcaagtcagagagaagacagctgtggatatgggtgaaaattgggttgga
gatgaaagaaggaagttcaatgggcacgctgaatttgagcctgtggagcgaggtggatttgggttaagaataagaatgagggctggagagatg
gctcagcggttaagggcactgactgctctgccagaggcagtgagttcaattcccagctggtggctcacaaccatctgtgatgggatccagtgcat
gaatgggaatgaggcgggaaagctggtggctcacaaccatctgtgatgggatccagtgcatgaatgggaatgaggcgggaaagctggtgga
gatgccagcacaagatgtgactgacaggttgtatggatggcaacggaagggacagaatgaagctgtgggtggagggagtgggttgaggaga
tgagtatggggccaaggagacaggactgacctcacaatgatagaagaaggcaagaataaatccaaggagcctagtgatatggctcaggagag
aaaggctcttgctgccaaagctaacaacctgagttaaatccctggagcccacacggtaggagagaaccaatttcaggaagtcgtcctctgatgtt
catagacatgtgcacaggtacacacatacacatgctataaatagatgtgacataatactttttgttgttgttttggctctttgagacaagcatcactattt
gccctggctgttctggaactccctaagtagaccaggctgacctctgactcacagaggtcctcctatatctgcctgctgagtgctgggattagatgt
gtgtgctgccatgcatgaggtaataaaaaatttagctggtagtggtggcacacaccggtaggatttgctgaaggaggcagaggcaggaggatc
acgagttcgaggccagcctaggctacagagtgagttccaggacagccagggctacacagagaaaccctgtctcaaaaaaaaaaaaaaaaa
aaaaaaaaaaaaaaaaaatttaaagtgtaaacttgtgaatgaaatggaagctagaataaaaaagagacccatataaagatggcactcaatttggtgt
tttcccccttctcttcaataggactctgagagactctgcttcctcgctaccacacttcggcccttccctgccatgctgggagggctggggacccagg
ggacctggaccagctcagagagggagcagctgtgggccatgaggctggatctccgggacctgcacaggcacctccgctttcaggtagagtg
cgcactgtacccttgaccccggggatctacaagcaaactggactcactggctctcttttgaaacacctcccagaatcaggttgaagaagcctagt
gagacacttcagcttctgtgagtgagtagcggccctagagcctggctctgcccaaagtctctgcctgctcttgagggctggactccagtgaaaga
atagccatgggacaccagggcagcccctgtcgtcatcgggcccagtatccgtcccttctccctccagttccgacttcactcggattctccgaggc
gggtcaaagggcaatagaaattttaccttagtaaaaagaaaattgaaaatgcaaaacaggaactacctgtgtcaccccctctgatcctgaaaaatc
cttaacagcagagaatttcctgaccagtgatccccagagcaaagcagtcccacccagtttggagcatcttgctctaccacgaccatttttaaaaata
gatagacagacagacagacagacaggtaagggggctggagagaaagtcccgtcgtttacagcactggctgctcttcccgaggacttgggtcc
 aatccccagcaactcaaccatttcacaaccatctgtaactccagttctagggtatctgacgccttttctctgctccttggcacttggtaagtggtgcac atacacacaggcagaacactcatacacataaaataaatttaatagttttttttttttaaatctagtacgccaagtgtggtgatgcattcctgtcgtctcaa acacttggttggttgaggcaggagaattgtgatttcaaggccacactgtaactatagaggaagattctgtaaaataaacaaacacaagaaacaga gctggagagatggcaagagcagcacttgctggtcttgcagaggataaatgctaacacccagcactctctctggccttgaagaacaccagtcata caggcagtgcacatacatacatgcagacaaagggctcgtatatacgaaataaaaaaataaatcatatatatatatgacagctcagcagtcaagaa cacagtttgtcttacaaaagacccaagtttgagtcccagcatccacatagtgactcacaaccatcctcaaatccagttccaggggacctgatgtctc gtctcacccccttaagcactgcacacatgtggtacacacccatacacattaaataaataaataaataaataataggaatgttaaagggaaaagaaa taaaatgcaagatttaaataaagacatgactagattagataaagactttgaccctgtaagtctgagggagaggctaccagagcaaccagggcctg tggttactgatgtcactggaggtgatgggcagcagaaggaatggagactctctctggttcttgatgtggccctgtgtgtctttcctcataggtgctg gctgcaggattcaaatgttcaaaggaggaggaggacaaggaggaagaggaagaggaggaagaagaagaaaagaagctgcccctaggggc tctgggtggccccaatcaggtgtcatcccaagtgtcctggccccagctgctctatacctaccagctccttcactccctggagcttgtcctgtctcgg gctgttcgggacctgctgctgctgtccctgcccaggcgcccaggctcagcctgggattcctaacacctagcttcaagccctatggagtgaccttc cagctccctccctcgcccgttaagactctaaggctggagtctggccaatcacaggacaggctctagctcgtttgccttagaccaggcagggcttc actagctcccagccctgacccaataatttaaaagccctccagtccttaccagatatttatttcttggatatttatttatttttaagaaatggtttatttattgtt tcactcttgagttaggccaccatgctgggtgcctaataaagccatccagcccgg. The nucleotide sequence set forth in SEQ ID NO: 2 is a genomic sequence encoding the P28 subunit.

In addition, a recombinant expression plasmid, a viral vector, or an mRNA fragment based on the recombinant IL-27 protein has any one of the following features:
A, including the recombinant IL-27 protein;
B, including a nucleotide sequence encoding the recombinant IL-27 protein; and
C, including a fragment with a nucleotide sequence encoding the recombinant IL-27 protein.

Further, a liposome preparation of the recombinant expression plasmid, the viral vector, or the mRNA fragment expressing the recombinant IL-27 protein is prepared by subjecting a nucleic acid preparation encoding the recombinant IL-27 protein to liposome modification.

Further, the recombinant expression plasmid or the viral vector is used to express the recombinant IL-27 protein in *Escherichia coli,* an insect cell, and a mammalian cell and *in vivo.*

Furthermore, a preparation method of a recombinant protein expressed in HEK293 cells based on the recombinant expression plasmid or the viral vector includes the following steps:
S1, directionally cloning a gene fragment with a nucleotide sequence set forth in SEQ ID NO: 2 into a pTriEx-1.1Hygro expression vector to obtain a recombinant expression plasmid;
S2, subjecting the recombinant expression plasmid to transient transfection into the HEK293 cells to express the recombinant protein; and
S3, separating and purifying the recombinant protein to obtain a pure recombinant protein.

The pTriEx-1.1Hygro is a two-hybrid plasmid used for protein expression. The pTriEx-1.1Hygro is an expression vector that can efficiently express recombinant proteins in *Escherichia coli.* The expression vector includes multiple cloning sites and selection markers, including a T7 RNA polymerase promoter, a lac operator, a His6 tag, and a TEV sequence. In addition, the pTriEx-1.1Hygro also has antibiotic resistance genes and hybridin resistance genes, and can be screened and expanded under a variety of different culture conditions.

The HEK293 cells are a human embryonic kidney cell line with high quality and stability. These cells are easy to culture, amplified, and transfect *in vitro* and can express a variety of proteins, including complex recombinant proteins and antibodies.

The transient transfection is a method of introducing exogenous DNA or RNA into cells. This method is a non-stable transfection technology, which generally enables the target cells to express exogenous proteins or RNA quickly and efficiently. The transient transfection is different from stable transfection, which requires the exogenous DNA or RNA to be integrated into a genome to achieve long-term expression.

Furthermore, the S2 of subjecting the recombinant expression plasmid to transient transfection into the HEK293 cells to express the recombinant protein includes:
S21, mixing the recombinant expression plasmid and a liposome transfection reagent; and
S22, transfecting an obtained mixture into HEK293 mammalian cells with a cell density of 50% to 80%, and culturing in an incubator at 37°C with 5% CO₂ for 48 h, 72 h, and 96 h, thereby transiently expressing the recombinant protein with a 6×His tag fused on a C-terminal.

The transfected cells are cultured for different time periods, namely 48 h, 72 h and 96 h, in order to optimize expression efficiency and yield of the recombinant protein. Different proteins require different expression times to reach maximum expression levels.

In the case of transient transfection, since the exogenous DNA/RNA is not integrated into the host genome, there is a highly short expression time.

Therefore, collecting and analyzing expressed cells at different time points can determine an optimal expression time point, thereby obtaining the recombinant protein with higher expression levels and purity. In this method, the recombinant protein is fused with a 6×His tag at the C-terminal, and thus can be separated and purified using affinity chromatography (such as nickel ion affinity chromatography).

The preparation method provided in this example aims to prepare the desired recombinant IL-27 protein. This method adopts transient transfection, which enables rapid expression and high yield, and the desired recombinant protein to be obtained in a short time. The construction with HEK293 cells has desirable stability and scalability, and can maintain a high expression level during long-term culture. Since the recombinant protein has a C-terminal-fused 6×His tag, simple separation and purification such as affinity chromatography can be conducted to improve purity and yield of the product.

In addition, compared with other production methods, the preparation method provided in the present disclosure does not require large-scale cell culture or expensive equipment and media, thereby reducing production costs.

In summary, the preparation method has the advantages of high efficiency, stability, easy purification, and economy, and can be widely used in the preparation and production of recombinant proteins.

In addition, a host cell is based on the recombinant IL-27 protein; and the host cell includes the recombinant expression plasmid.

The host cell refers to a vector used for gene expression in a recombinant DNA or RNA expression system, and may be a known cell line or strain. In the present discloure, an exogenous gene (recombinant expression plasmid in the present disclosure) is introduced into the host cell to produce a desired protein or RNA product through metabolism and mechanism.

Further, in the application of the present disclosure, a cloned plasmid based on the recombinant IL-27 protein is provided; and the cloned plasmid has any one of the following features:
A, including the recombinant IL-27 protein;
B, including a nucleotide sequence encoding the recombinant IL-27 protein; and
C, including a fragment with a nucleotide sequence encoding the recombinant IL-27 protein.

Further, in the application of the present disclosure, the product is any one selected from the group consisting of a drug, a detection reagent, a kit, and a diagnostic reagent.

Further, the drug includes the IL-27 protein and a pharmaceutically acceptable carrier; the pharmaceutically acceptable carrier is at least one selected from the group consisting of a sustained-release agent, an excipient, a filler, a binder, a wetting agent, a disintegrant, an absorption promoter, a surfactant, and a lubricant; and the drug has a dosage form selected from the group consisting of an ointment, a powder, a tablet, a capsule, and a granule.

The detection reagent, kit, and diagnostic reagent can be used to detect a receptor-based IL-27Ra agonist drug. Moreover, the product can also be a detection apparatus, detection device, and a detection system.

Further, the present disclosure provides use of an IL-27 protein in treating and/or delaying AD; where the IL-27 protein is a recombinant IL-27 protein targeting a therapeutic target IL-27; and the IL-27 protein includes a mammal-derived IL-27 protein.

In the present disclosure, the IL-27 protein includes preferably a mouse-derived IL-27 protein and/or a human-derived IL-27 protein.

In the present disclosure, an administration method of the IL-27 protein includes injection; the injection includes intracranial injection; and a dosage of the IL-27 protein is 100 ng/side in a mouse by the intracranial injection.

Furthermore, the present disclosure provides a method for treating AD, including the following steps:
administering the IL-27 protein to an AD patient; where the IL-27 protein is a recombinant IL-27 protein targeting a therapeutic target IL-27; and the IL-27 protein includes a mammal-derived IL-27 protein.

In the present disclosure, the IL-27 protein includes a mouse-derived IL-27 protein and/or a human-derived IL-27 protein.

The present disclosure will be further illustrated below through specific examples. However, it should be understood that these examples are for the purpose of more detailed illustration only, and should not be understood as being intended to limit the present disclosure in any way.

**Example 1:** in this example, the specific high expression of IL-27 receptor in mouse hippocampus was verified by experiments.
1. Experimental methods: (1) C57BL/6J wild-type male mice (8 weeks old, Beijing Vital River Laboratory Animal Technology Co., Ltd.) were housed in the Laboratory Animal Center of Jinan University. The mice were maintained in a feeding room under constant temperature and humidity with a 12 h light-dark cycle. The mice had free access to feed and water. All animal procedures were conducted in accordance with procedures approved by the Animal Welfare Ethics Committee. (2) The hippocampal tissue of mice was obtained for *in situ* hybridization detection of IL-27 receptor gene.
2. Experimental results:

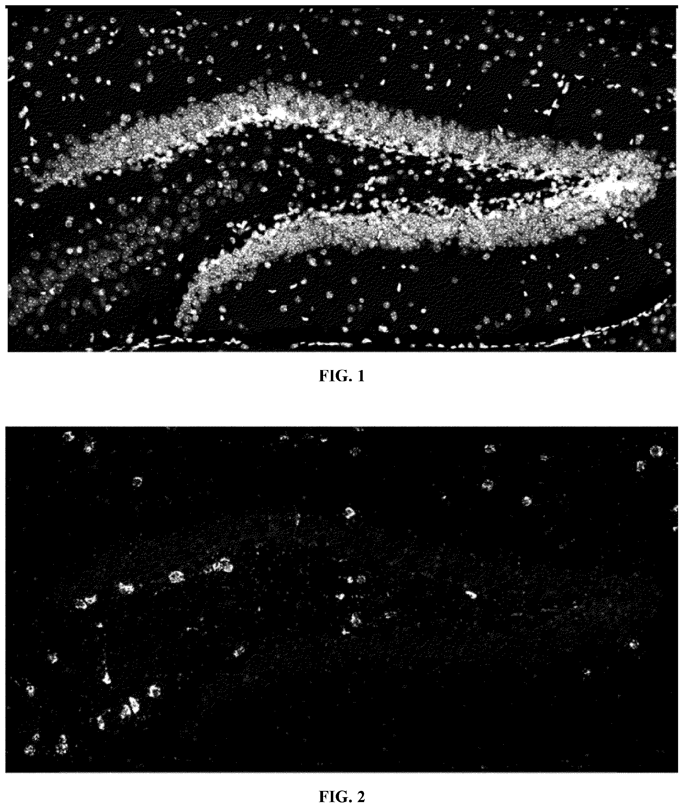

As shown in FIG. 1 to FIG. 5, FIG. 1 to FIG. 5 showed the expression of IL-27Ra in hippocampal tissue. After the *in situ* hybridization detection of IL-27 receptor gene in C57BL/6J experimental mice, FIG. 1 to FIG. 5 were immunohistochemical staining images of DAPI, IL27Ra, VGlut, Vgat, and Merge, respectively.

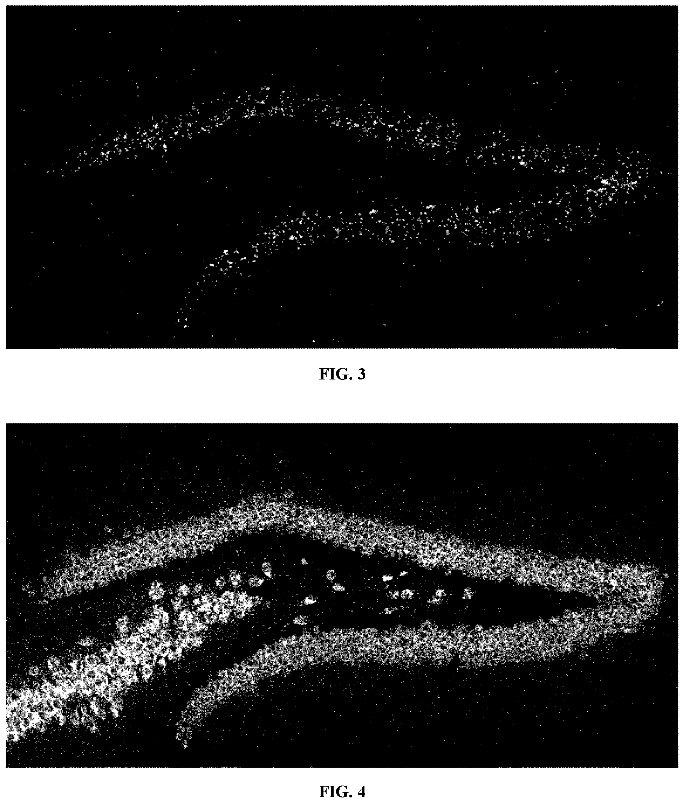

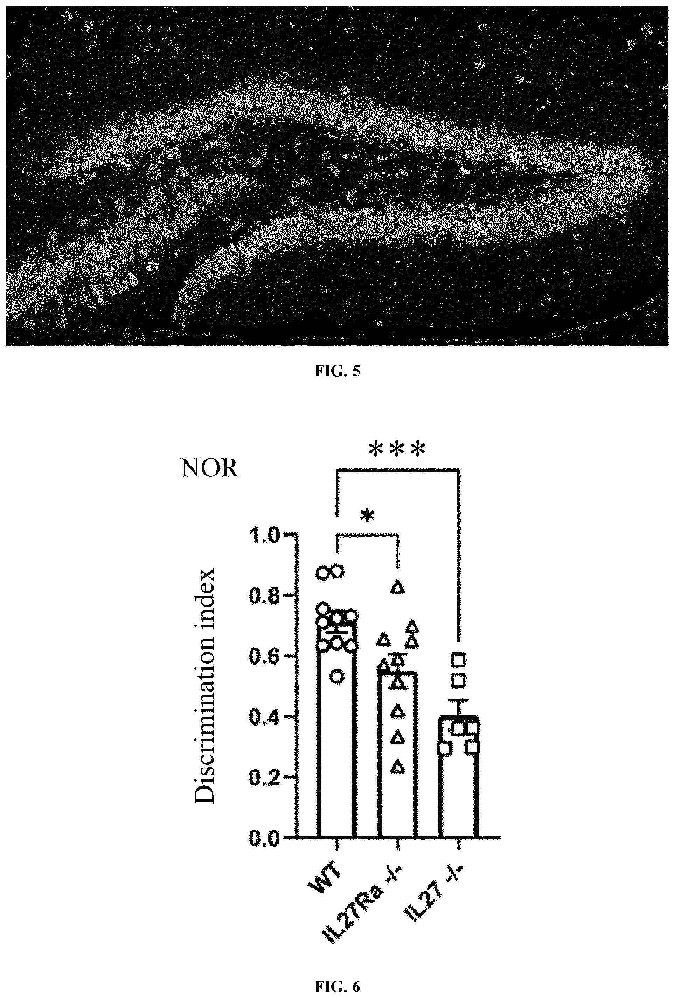

In FIG. 1, DAPI was a DNA stain that could stain DNA in the cell nucleus and showed blue fluorescence. In FIG. 2, IL27RA was the abbreviation of interleukin 27 receptor α subunit, which was the molecule being detected; in this figure, IL27RA was immunohistochemically stained with a specific antibody, showing a green fluorescence signal. In FIG. 3, VGlut was a glutamate transporter, which was mainly present in glutamatergic neurons; in this figure, VGlut was immunohistochemically stained with a specific antibody, showing a red fluorescence signal. In FIG. 4, Vgat was a GABA transporter, which was mainly present in GABAergic neuron, where Vgat was immunohistochemically stained with a specific antibody, showing a yellow fluorescence signal. In FIG. 5, Merge referred to a color image formed by merging all signals together to show the relationship and overlap between different markers.

Through the *in situ* hybridization detection, it was found that IL-27 receptor was specifically highly expressed in the dentate gyrus region of mouse hippocampus; further analysis of neuronal subtypes revealed that the IL-27 receptor was mainly expressed in glutamatergic neurons.

**Example 2:** in this example, experiments demonstrated that IL-27 gene-deficient mice and IL-27 receptor gene-deficient mice exhibited significant impairment of learning and memory abilities.
1. Experimental method: the experiment was set up with IL-27KO and IL-27RaKO female mice (gene-deficient mice, purchased from Jaxlab, USA), with at least 6 mice in each group. Behavioral tests of learning and memory behaviors such as NOR, NOL, and Barnes maze tests were conducted on 6-8 week old mice.
2. Experimental results:

The results were shown in FIG. 6 to FIG. 9.

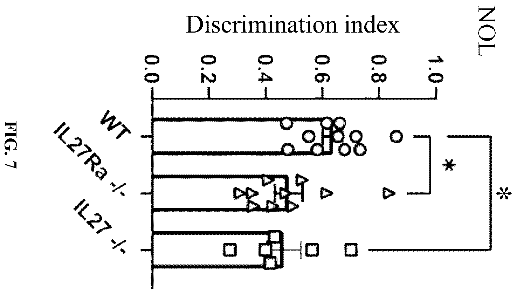

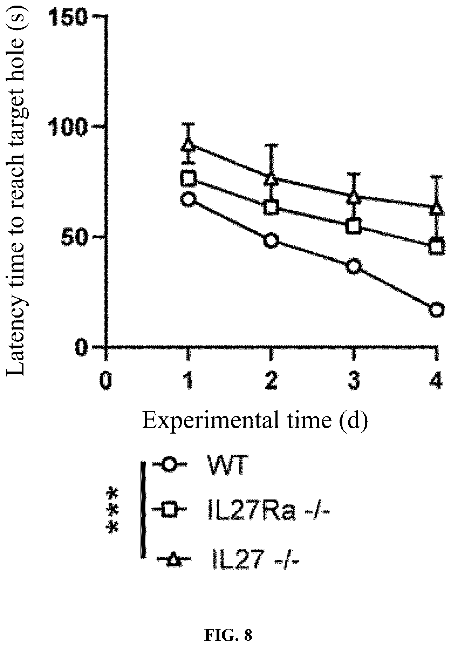

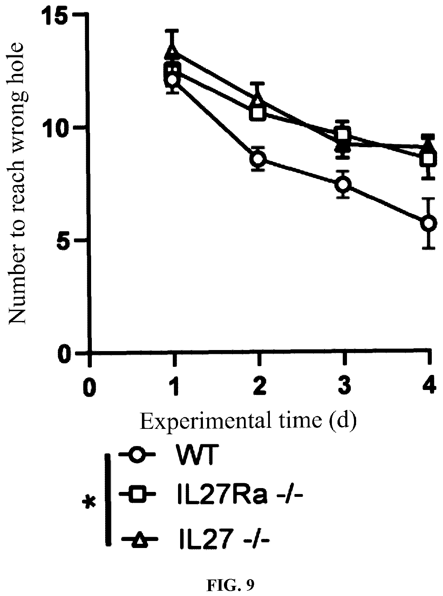

FIG. 6 to FIG. 9 were all behavioral detection experiments. FIG. 6 and FIG. 7 showed the discrimination index in NOR; FIG. 7 showed the discrimination index in NOL of gene-deficient mice; FIG. 8 showed a latency time for gene-deficient mice to reach the target hole in the Barnes maze test; FIG. 9 showed a number of times that gene-deficient mice entered the wrong hole in the Barnes maze test.

As shown in FIG. 6 to FIG. 9, the detection time of IL-27KO mice and IL-27RaKO mice for novel and old objects and novel and old locations was similar compared with the WT group, and the KO mice had obvious memory impairment. In addition, in the Barnes maze test, the latency time of IL-27KO mice and IL-27RaKO mice to reach the target box was also prolonged compared with the WT group.

The above experimental results showed that the IL-27 signaling pathway was crucial for maintaining learning and memory abilities.

**Example 3:** in this example, an experiment was conducted to show that IL-27 improved the severity of AD in FADSX AD experimental mice.

### 1. Experimental method:

(1) FAD5X AD female mice (36 weeks old, Nanjing GemPharmatech Co., Ltd.) were randomly divided into:
   A, a control group (corresponding to FAD 5X+ACSF in the figure); and
   B, an Rm IL-27 group (corresponding to FAD 5X+IL27 in the figure); where

There were at least 7 mice in each group.

Drug administration was conducted in the designated hippocampus. Number of doses: 1 time.

Specifically:
A, control group: the mice were injected with an equal volume of artificial cerebrospinal fluid (ACSF) per day; and
B, Rm IL-27 group: the mice were intracranial injected with the recombinant IL-27 protein (dissolved in sterile water) at 100 ng/side (one side each of the left and right brain).

(2) The mice were subjected to behavioral tests of NOR, NOL, and Barnes maze tests at two time points, 1 h and 24 h after administration.

### 2. Experimental results:

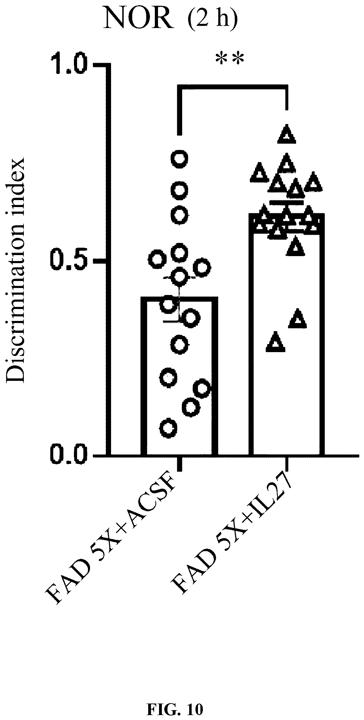

The results were shown in FIG. 10 to FIG. 14.

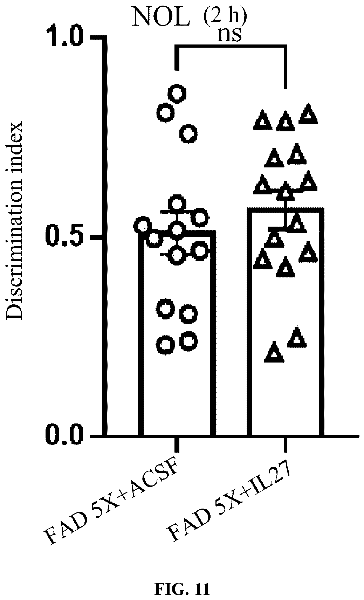

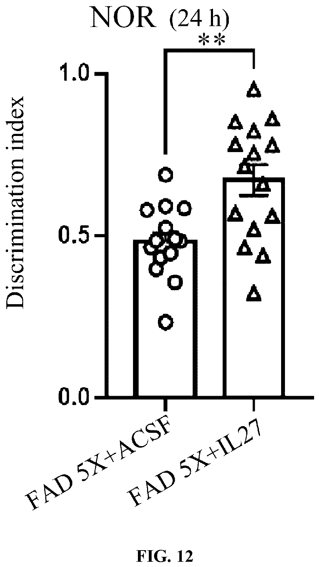

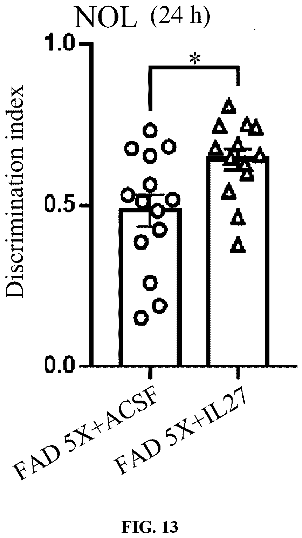

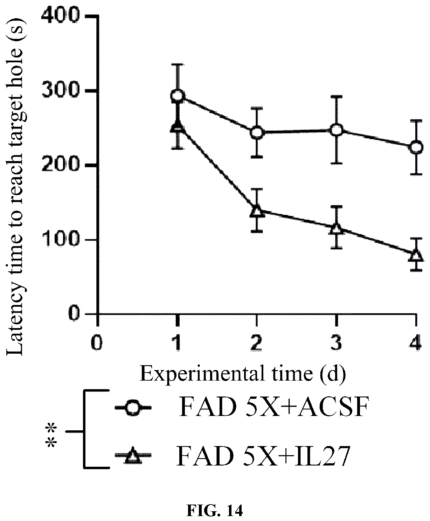

FIG. 10 to FIG. 14 were all behavioral detection experiments. FIG. 10 showed a comparison of discrimination indexes of FAD5X AD (FAD) experimental mice (RM IL-27 group and control group) in the novel object recognition (NOR) test at a 2 h time point; FIG. 11 showed a comparison of discrimination indexes of FAD5X AD experimental mice (RM IL-27 group and control group) in the novel object location (NOR) test at a 2 h time point; FIG. 12 showed a comparison of discrimination indexes of FAD5X AD experimental mice (RM IL-27 group and control group) in the novel object recognition (NOR) test at a 24 h time point; FIG. 13 showed a comparison of discrimination indexes of FAD5X AD experimental mice (RM IL-27 group and control group) in the novel object location (NOR) test at a 24 h time point; FIG. 14 showed a latency time of the FAD5X AD experimental mice (RM IL-27 group and control group) to reach a target hole in a Barnes maze test.

FIG. 10 to FIG. 14 as a whole could show the effect of IL-27 on experimental AD-psoriasis. As shown in FIG. 10 to FIG. 14, the sniffing time of the IL-27 group to novel objects and novel locations was significantly increased compared with the control group; the latency time of mice reaching the target box in the Barnes maze test was significantly shortened. This suggested that IL-27 delayed and treated the progression of AD in experimental mice.

**Example 4:** in this example, an experiment was conducted to show that IL-27-mRNA improved the severity of AD in FAD5X AD experimental mice.

### 1. Experimental method:

(1) FAD5X AD female mice (36 weeks old, Nanjing GemPharmatech Co., Ltd.) were randomly divided into:
   A, a control group (corresponding to +ACSF in the figure, namely FAD 5X+ACSF); and
   B, an IL-27-mRNA group (corresponding to +IL27mRNA in the figure, namely FAD5X+IL27mRNA).

There were at least 7 mice in each group.

Targeted administration was conducted at hippocampus. Number of administration: 1 time.

Specifically:
A, control group: the mice were injected with an equal volume ofACSF per day; and
B, IL-27-mRNA group: the mice were intracranially injected with IL-27-mRNA liposome nanoparticles (LNPs) (dissolved in sterile water) at 100 ng/side.

(2) The mice were subjected to behavioral tests of NOR, NOL, and Barnes maze tests at two time points, 36 h and 72 h after administration.

### 2. Experimental results:

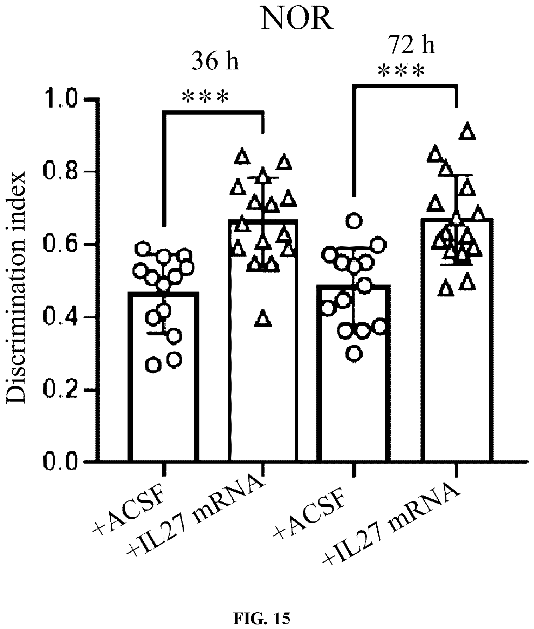

The results were shown in FIG. 15 to FIG. 17.

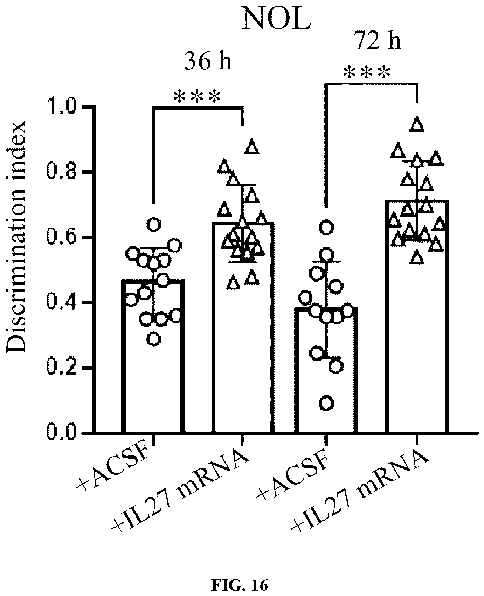

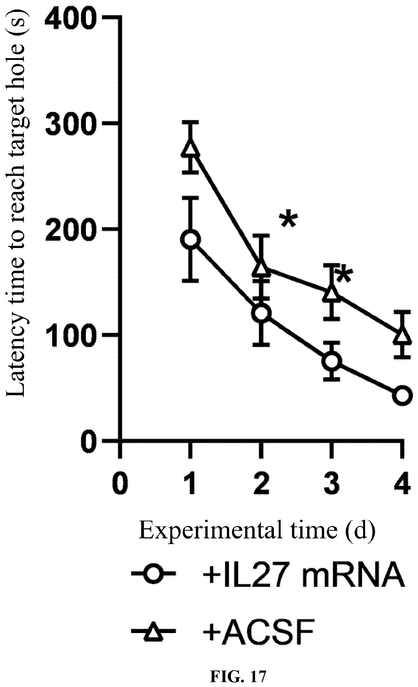

FIG. 15 to FIG. 17 were all behavioral detection experiments. FIG. 15 showed a comparison of discrimination indexes of FAD5X AD (FAD) experimental mice (IL-27-mRNA group and control group) in the NOR test at 36 h after drug administration and at 72 h after drug administration; FIG. 16 showed a comparison of discrimination indexes of FAD5X AD (FAD) experimental mice (IL-27-mRNA group and control group) in the NOL test at 36 h after drug administration and at 72 h after drug administration; and FIG. 17 showed a latency time of the FAD5X AD (FAD) experimental mice (IL-27-mRNA group and control group) to reach a target hole in a Barnes maze test.

FIG. 15 to FIG. 17 showed the influence of IL-27-mRNA on experimental AD. As shown in FIG. 15 to FIG. 17, the sniffing time of the mRNA-IL-27 group to new objects and new locations was significantly increased compared with the control group; the latency time of mice reaching the target box in the Barnes maze test was significantly shortened.

Experimental results suggested that the IL-27-mRNA delivery system delayed and treated the progression of AD in experimental mice.

The above are the preferred embodiments and corresponding examples of the present disclosure. It should be pointed out that for those of ordinary skilled in the art without departing from the creative concept of the present disclosure, several modifications and improvements can be made, including but not limited to the adjustment of proportions, processes, dosages and reaction vessels, such as the use of continuous flow reactors. These are all within the scope of protection of the present disclosure. The above are the preferred embodiments and corresponding examples of the present disclosure. It should be pointed out that for those of ordinary skilled in the art, without departing from the creative concept of the present disclosure, several modifications and improvements can be made, including but not limited to the adjustment of proportions, processes, and dosages. These are all within the scope of protection of the present disclosure.

## Claims

1. Use of an interleukin 27 (IL-27) protein in preparation of a product for treating and/or delaying Alzheimer's disease (AD), wherein the IL-27 protein is a recombinant IL-27 protein targeting a therapeutic target IL-27; and
the IL-27 protein comprises a mammal-derived IL-27 protein.

2. The use according to claim 1, wherein the IL-27 protein comprises a mouse-derived IL-27 protein and/or a human-derived IL-27 protein.

3. The use according to claim 1, wherein the recombinant IL-27 protein is any one selected from the group consisting of:
A, a protein with an amino acid sequence set forth in SEQ ID NO: 1; and
B, a protein derived from the amino acid sequence set forth in SEQ ID NO: 1 or a mammalian homologous protein sequence thereof, wherein the protein is prepared by subjecting the amino acid sequence set forth in SEQ ID NO: 1 to substitution, deletion, or addition of one or more amino acid residues and has a same activity.

4. The use according to claim 3, wherein a gene encoding the recombinant IL-27 protein comprises:
a nucleotide sequence set forth in SEQ ID NO: 2; or a nucleotide sequence encoding a protein with a same activity and prepared by subjecting the nucleotide sequence set forth in SEQ ID NO: 2 to substitution, deletion, and/or addition of one or more nucleotides; or a nucleotide sequence encoding the amino acid sequence set forth in SEQ ID NO: 1.

5. The use according to claim 1, wherein a recombinant expression plasmid, a viral vector, or an mRNA fragment expressing the recombinant IL-27 protein has any one of the following features:
B, comprising a nucleotide sequence encoding the recombinant IL-27 protein; and
C, comprising a fragment with a nucleotide sequence encoding the recombinant IL-27 protein.

6. The use according to claim 5, wherein a liposome preparation of the recombinant expression plasmid, the viral vector, or the mRNA fragment expressing the recombinant IL-27 protein is prepared by subjecting a nucleic acid preparation encoding the recombinant IL-27 protein to liposome modification.

7. The use according to claim 5, wherein the recombinant expression plasmid or the viral vector expressing the recombinant IL-27 protein is used to express the recombinant IL-27 protein in *Escherichia coli,* an insect cell, and a mammalian cell and *in vivo.*

8. The use according to claim 5, wherein a host cell expressing the recombinant IL-27 protein comprises the recombinant expression plasmid.

9. The use according to claim 1, wherein a cloned plasmid encodes the recombinant IL-27 protein; and the cloned plasmid has any one of the following features:
B, comprising a nucleotide sequence encoding the recombinant IL-27 protein; and
C, comprising a fragment with a nucleotide sequence encoding the recombinant IL-27 protein.

10. The use according to claim 1, wherein the product is any one selected from the group consisting of a drug, a detection reagent, a kit, a diagnostic reagent, detection apparatus, a detection device, or a detection system.

11. The use according to claim 10, wherein the drug comprises the IL-27 protein and a pharmaceutically acceptable carrier;
the pharmaceutically acceptable carrier is at least one selected from the group consisting of a sustained-release agent, an excipient, a filler, a binder, a wetting agent, a disintegrant, an absorption promoter, a surfactant, and a lubricant; and
a dosage form of the drug is selected from the group consisting of an ointment, a powder, a tablet, a capsule, and a granule.

12. Use of an IL-27 protein in treating and/or delaying AD; wherein the IL-27 protein is a recombinant IL-27 protein targeting a therapeutic target IL-27; and
the IL-27 protein comprises a mammal-derived IL-27 protein.

13. The use according to claim 12, wherein the IL-27 protein comprises a mouse-derived IL-27 protein and/or a human-derived IL-27 protein.

14. The use according to claim 12, wherein an administration method of the IL-27 protein comprises injection.

15. The use according to claim 14, wherein the injection comprises intracranial injection.

16. The use according to claim 15, wherein a dosage of the IL-27 protein is 100 ng/side in a mouse by the intracranial injection.
